# EUROPÄISCHE PATENTSCHRIFT

(11) **EP 1 300 176 B1**
(45) Veröffentlichungstag und Bekanntmachung des Hinweises auf die Patenterteilung: **02.11.2006**
(21) Anmeldenummer: 02020148.9
(22) Anmeldetag: 09.09.2002
(51) Int. Cl.: A61N 5/06

(54) **Bestrahlungsgerät, insbesondere zur fotodynamischen Therapie**
Irradiation device, particularly for photodynamic therapy
Dispositif d'irradiation, en particulier pour la thérapie photodynamique

(30) Priorität: 08.10.2001 DE 10149462
(43) Veröffentlichungstag der Anmeldung: 09.04.2003
(73) Patentinhaber: Herbert Waldmann GmbH & Co. KG, 78056 Villingen-Schwenningen (DE)
(72) Erfinder: Waldmann, Gerhard, 78083 Dauchingen (DE)
(74) Vertreter: Patentanwälte Westphal, Mussgnug & Partner

(56) Entgegenhaltungen:
- EP-A- 1 138 349
- WO-A-93/09847
- DE-U- 8 813 852
- US-A- 4 068 156

## Beschreibung

Die Erfindung betrifft ein Bestrahlungsgerät zur fotodynamischen Therapie mit einem mit Strahlenquellen ausgerüsteten Bestrahlungskopf.

Es sind Bestrahlungsgeräte z. B. für die fotodynamische Therapie bekannt, die eine Bestrahlung des Kopfes und der Extremitäten eines Patienten von drei Seiten erlauben. Zur flächigen Bestrahlung des Kopfes, Rumpfes oder der Extremitäten eines Patienten sind Bestrahlungsgeräte anderer Bauart vorgesehen.

Ein Bestrahlungsgerät, das sowohl für eine dreiseitige als auch eine flächige Bestrahlung geeignet ist, ist aus der WO 93109847 bekannt.

Die Erfindung wird durch die im Anspruch 1 angegebenen Merkmale definiert.

Der Bestrahlungskopf des erfindungsgemäßen Bestrahlungsgerätes besteht aus einem Mittelteil und zwei Seitenflügeln, die an zwei gegenüberliegenden Seiten des Mittelteils seitlich schwenkbar befestigt sind. Der Bestrahlungskopf läßt sich zu einer ebenen Fläche aus dem Mittelteil und den beiden mit dem Mittelteil in einer Ebene liegenden Seitenflügeln formen. Durch Schwenken der Seitenflügel kann die ebene Fläche stufenlos bis zu einem rechtwinkligen U verstellt werden.

Das Mittelteil des Bestrahlungskopfes ist z. B. mittels eines U-förmigen Bügels an einem am einen Ende eines Schwenkarmes angeordneten ersten Drehgelenk drehbar befestigt. Die Drehebene liegt parallel zur Fläche des Mittelteils.

Das andere Ende des Schwenkarmes, der z. B. teleskopartig einund ausfahrbar gestaltet sein kann, ist mittels eines zweiten Drehgelenkes drehbar an einer Säule befestigt, die in einem Säulenhalter verschiebbar geführt und feststellbar ist. Die Drehachse des Mittelteils des Bestrahlungskopfes steht senkrecht zur Drehachse des Schwenkarmes.

Der Säulenhalter ist an einem Schaltschrank befestigt.

Bei einem Ausführungsbeispiel der Erfindung ist auf der Oberseite des Schaltschrankes eine Bedienkonsole vorgesehen.

Ein weiteres Ausführungsbeispiel sieht einen schwenkbaren Ausleger an der Schaltkonsole vor, um ihr einen kippsicheren Halt zu geben. Die Schaltkonsole kann z. B. mit Rollen oder Rädern ausgerüstet sein, um das erfindungsgemäße Bestrahlungsgerät schnell und einfach an den jeweiligen Einsatzort verbringen zu können.

Die Säule ist um ihre Längsachse drehbar im Säulenhalter geführt.

Das Mittelteil und die Seitenflügel des Bestrahlungskopfes sind vorzugsweise als rechteckförmige Platten ausgeführt, an oder in deren Unterseiten Strahlenquellen wie z. B. Leuchtmittel angeordnet sind. Als Leuchtmittel kommen z. B. Stab- oder Kompaktleuchten in Frage. Vorzugsweise sind die Strahlenquellen austauschbar. Die Leuchtmittel weisen ein Spektrum von Ultraviolett über das sichtbare Licht bis hin zu Infrarot auf. Zur Kühlung der Strahlenquellen bzw. der Leuchtmittel ist vorzugsweise eine Kühlvorrichtung vorgesehen, z.B eine Zwangsentlüftung oder Lüfter.

Am Befestigungsende der Seitenflügel sind z. B. halbkreisförmige Zahnsegmente vorgesehen, die in einer am Mittelteil angeordneten Achse drehbar angeordnet sind. Zum Verstellen der Seitenflügel ist z. B. weiter eine Welle mit je einer Schnecke am Wellenende vorgesehen. Die Schnecken sind gegenläufig und kämmen die Zahnsegmente der Seitenflügel. Die Welle kann beispielsweise von einem Elektromotor angetrieben werden.

Alternativ hierzu ist eine manuelle Verstellung der Seitenflügel möglich. Die Seitenflügel sind in einer Achse des Mittelteils gelagert und mittels eines Stiftes in vorgebbaren Stellungen feststellbar.

Das Drehgelenk, mit welchem das Mittelteil des Bestrahlungskopfes am einen Ende des Schwenkarmes befestigt ist, besteht im Wesentlichen aus zwei aufeinanderliegenden Gelenkteilen, wobei zur Zentrierung eines der beiden Gelenksteile einen Ansatz aufweist, der in einer korrespondierenden Aussparung des anderen Gelenkteiles drehbar gelagert ist. Einzelheiten dieses Drehgelenkes sind Gegenstand der Ansprüche 8 bis 11.

Die elektrischen Leitungen zur Stromversorgung der Strahlenquellen bzw. Leuchten sind zentral durch das erste Gelenk geführt.

Weil der Bestrahlungskopf von einer ebenen Fläche stufenlos oder stufenweise bis zu einem rechtwinkligen U verformbar ist, erlaubt das erfindungsgemäße Bestrahlungsgerät sowohl eine flächenhafte als auch eine dreiseitige Bestrahlung. Weil der Bestrahlungskopf außerdem um zwei senkrecht zueinander stehende Achsen drehbar und mittels der Säule in seiner Höhe verstellbar ist, läßt sich der Bestrahlungskopf optimal an die individuelle Anatomie eines Patienten anpassen. Mittels des erfindungsgemäßen Bestrahlungsgerätes kann eine flächige Bestrahlung von Körperbereichen, wie z. B. des Kopfes, des Rumpfes und der Extremitäten, durchgeführt werden. Ebenso läßt sich eine dreiseitige Bestrahlung realisieren. Der Patient kann sich in Folge der Verstellmöglichkeiten des Bestrahlungskopfes in stehender, sitzender oder liegender Position befinden.

Die Erfindung wird anhand von in der Zeichnung dargestellten Einführungsbeispielen näher erläutert.

In der Zeichnung zeigen:
- Figur 1: ein Ausführungsbeispiel des erfindungsgemäßen Bestrahlungsgerätes in perspektivischer Ansicht,
- Figur 2: ein Ausführungsbeispiel eines Gelenkes zur Befestigung des Bestrahlungskopfes am Schwenkarm in Draufsicht,
- Figur 3: einen Längsschnitt durch das Gelenk aus Figur 2,
- Figur 4: ein Ausführungsbeispiel einer Verstellvorrichtung zum Verstellen der Seitenflügel des Bestrahlungskopfes in Seitenansicht und
- Figur 5: einen Ausschnitt eines Ausführungsbeispieles einer manuell betätigbaren Verstellvorrichtung zum Verstellen der Seitenflügel des Bestrahlungskopfes.

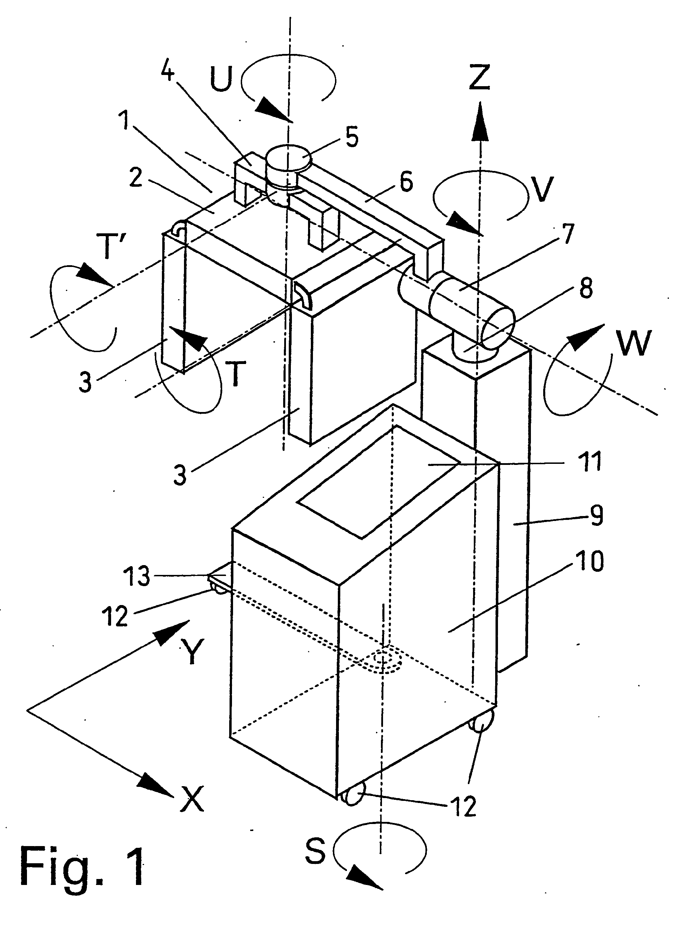

In Figur 1 ist ein Ausführungsbeispiel eines erfindungsgemäßen Bestrahlungsgerätes in perspektivischer Ansicht dargestellt.

Das Ausführungsbeispiel ist im Wesentlichen aus einem Bestrahlungskopf 1, einem Schwenkarm 6, einer Säule 8, einem Säulenhalter 9 und einem Schaltschrank 10 aufgebaut.

Der Bestrahlungskopf 1 besteht aus einem plattenförmigen Mittelteil 2 und zwei plattenförmigen Seitenflügeln 3, die an zwei gegenüberliegenden Seiten des Mittelteils 2 schwenkbar befestigt sind. Die Seitenflügel 3 sind um die Achsen T und T' schwenkbar. Sie können zusammen mit dem Mittelteil 2 eine ebene Fläche zur flächigen Bestrahlung bilden. Die Seitenflügel 3 lassen sich, wie bereits erwähnt, um die Achsen T und T' schwenken, so daß der Bestrahlungskopf 1 von einer ebenen Fläche in ein rechtwinkliges U verformt werden kann. Diese U-Form des Bestrahlungskopfes 1 ist besonders zur dreiseitigen Bestrahlung des Kopfes und der Extremitäten eines Patienten geeignet, während die ebene Flächenform zur flächigen Bestrahlung der Extremitäten oder des Rumpfes eines Patienten geeignet ist.

An oder in den Unterseiten des Mittelteils 2 und der Seitenflügel 3 sind die Strahlenquellen zur Bestrahlung eines Patienten vorgesehen. Als Strahlenquellen dienen beispielweise Stab- oder Kompaktleuchten, die vorzugsweise austauschbar sind. Diese Leuchtmittel umfassen ein Spektrum von Ultraviolett über das sichtbare Licht bis hin zu Infrarot. Der Übersichtlichkeit wegen sind die Leuchtmittel nicht in Figur 1 gezeigt.

Die Seitenflügel 3 sind entweder von Hand oder mittels eines Elektromotors schwenkbar.

Die Oberseite des Mittelteils 2 ist an den Schenkeln eines U-förmigen Bügels 4 angebracht, der mittels eines Drehgelenkes 5 an einem vorzugsweise teleskopartig ausziehbaren Schwenkarm 6 befestigt ist. Das dem Drehgelenk 5 gegenüberliegende Ende des Schwenkarmes 6 ist mittels eines Drehgelenkes 7 um 360° drehbar mit dem oberen Ende einer Säule 8 verbunden, die entlang ihrer Mittelachse Z verschiebbar und um 360° um ihre Mittel-achse Z in Richtung V drehbar in einem Säulenhalter 9 geführt ist. Die Säule 8 kann in beliebiger Stellung im Säulenhalter 9 arretiert werden, der an der Rückwand eines Schaltschrankes 10 befestigt ist. Alternativ hierzu kann der Säulenhalter 9 auch an einer Wand eines Behandlungsraumes befestigt sein. Der Schaltschrank 10, auf dessen Oberseite eine Bedienkonsole 11 vorgesehen ist, ist an seiner Unterseite mit Rollen oder Rädern 12, ausgerüstet, so dass er verfahrbar ist. Zur Erhöhung der Standsicherheit ist ein Ausleger 13 an der Unterseite des Schaltschrankes 10 vorgesehen, der z. B. in Richtung S drehbar oder ausziehbar an der Unterseite des Schaltschrankes vorgesehen ist.

Der Bestrahlungskopf 1 ist einerseits um die Achse U und die parallel hierzu verlaufende Achse Z drehbar und andererseits um die senkrecht dazu stehende Achse W drehbar. Außerdem ist der Bestrahlungskopf 1 in beliebiger Höhe einstellbar. Der Drehbereich um die Achsen U, Z und W kann 360° umfassen oder durch Anschlagmittel auf einen vorgebbaren Wert begrenzt sein.

Weil der Bestrahlungskopf 1 entlang der Achse Z höhenverstellbar, um die Drehachsen U, W und Z drehbar ist sowie seine Seitenflügel um die Achsen T und T' schwenkbar sind, wird eine optimale Anpassung an die individuelle Anatomie eines Patienten erzielt.

Die Leitungen zur Stromversorgung der Leuchtmittel im Mittelteil 2 und in den Seitenflügeln 3 führen vom Schaltschrank 10 zentral durch Drehgelenk 7, den Schwenkarm 6, das Drehgelenk 5 und den U-förmigen Bügel 4 zu den Leuchtmitteln.

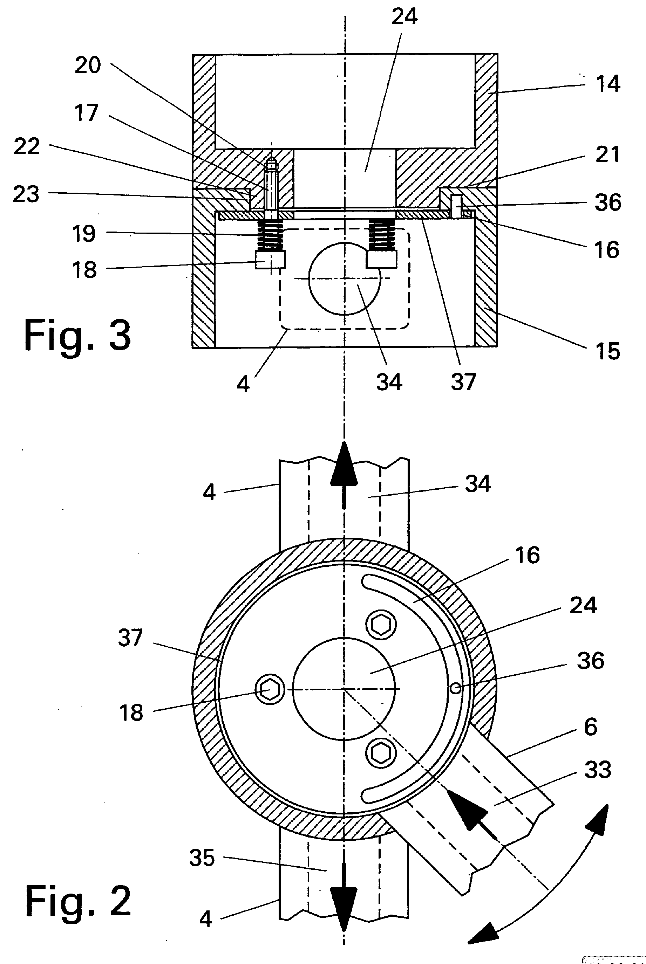

In Figur 2 ist das Drehgelenk 5 in Draufsicht gezeigt, während in Figur 3 ein Längsschnitt durch das Drehgelenk 5 dargestellt ist.

Das Drehgelenk 5 besteht aus einem oberen Gelenkteil 14 mit einem Ansatz 22 und einem unteren Gelenkteil 15 mit einer den Ansatz 22 aufnehmenden Aussparung 23. Mit dem Ansatz 22 ist eine Scheibe 37 mittels Schrauben 17 verbunden. Die Scheibe 37 ist so dimensioniert, daß ihre radial äußere, den Ansatz 22 überragende Ringfläche reibschlüssig an dem die Aussparung 23 des unteren Gelenkteiles 15 begrenzenden Kragenteiles anliegt. Hierdurch sind die Gelenkteile 14 und 15 axial fest aber in Unfangsrichtung drehbar miteinander verbunden. Die Verdrehbarkeit ist beschränkt durch einen Anschlagstift 36, welcher mit dem unteren Gelenkteil 15 verbunden ist und in eine kreissegmentförmige Aussparung 16 der Scheibe 37 eingreift.

Wie insbesondere Figur 3 erkennen läßt, sind zwischen den Schraubenköpfen 18 der Schrauben 17 Federelemente in Form von Druckfedern 19 angeordnet. Diese Ausgestaltung ermöglicht eine Veränderung der auf die Scheibe 37 wirkenden Anpreßkraft, wodurch das Reibmoment in gewünschter Weise eingestellt oder nachgestellt werden kann. Als Federelement kann anstelle der Schraubenfeder 19 auch eine Tellerfeder dienen.

Die Leitungen zur Stromversorgung der Leuchtmittel, welche der Übersichtlichkeit wegen nicht in den Figuren 2 und 3 gezeigt sind, sind durch die zentrale Öffnung 24 der beiden Gelenkteile 14 und 15 geführt.

Die Leitungen zur Stromversorgung der Leuchtmittel sind durch einen zentralen Kabelkanal 33 des Schwenkarmes 6, durch die zentralen Öffnungen 24 des oberen Gelenkteiles 14 und des unteren Gelenkteiles 15 sowie durch die zentralen Kabelkanäle 34 und 35 des U-förmigen Bügels 4 geführt. Die Pfeile deuten den Verlauf der Leitungen an.

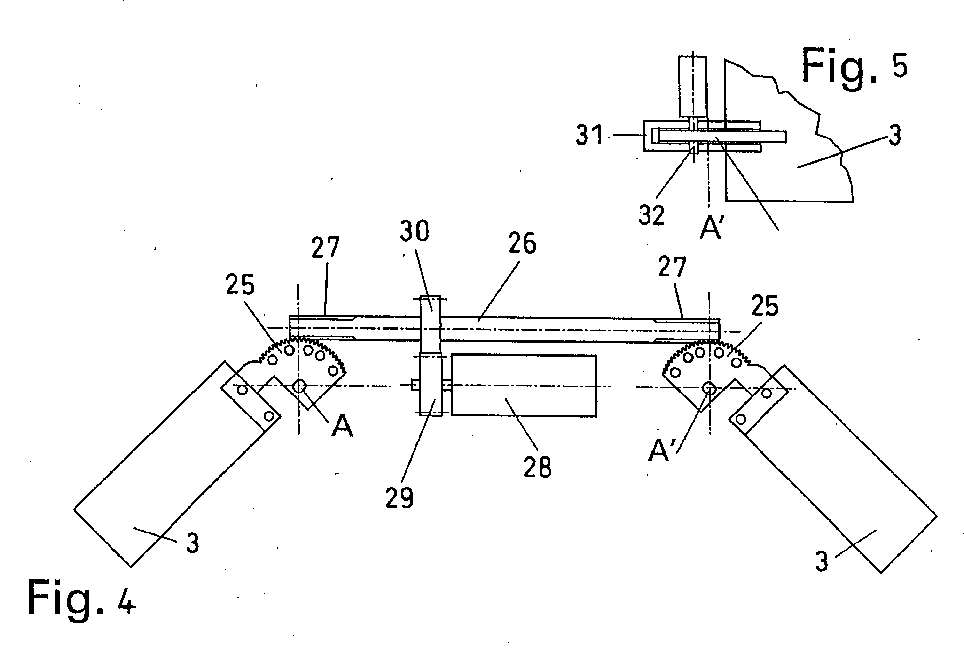

In Figur 4 ist die Verstellvorrichtung zum Verstellen der Seitenflügel des Bestrahlungskopfes in Seitenansicht gezeigt.

Am oberen Ende eines jeden Seitenflügels 3 ist ein viertelkreisförmiges Zahnsegment 25 vorgesehen, das um eine Achse A bzw. A' drehbar am Mittelteil 1 befestigt ist. Die Achsen A und A' entsprechen den Drehachsen T und T'. An jedem Ende einer Welle 26 ist eine Schnecke 27 eingearbeitet. Die eine Schnecke 27 kämmt mit dem einen Zahnsegment 25, während die andere Schnecke 27 mit dem anderen Zahnsegment 25 kämmt. Die beiden Schnecken 27 sind gegenläufig. Das Zahnrad 29 eines Elektromotors 28 treibt ein auf der Welle 26 sitzendes Zahnrad 30 an. Die Seitenflügel 3 können somit mittels des Elektromotors 28 verstellt werden.

In Figur 5 ist ein Ausschnitt einer manuellen Verstellvorrichtung zum Verstellen der Seitenflügel des Bestrahlungskopfes gezeigt.

Auf jeder Seite ist ein Seitenflügel 3 in einem Lager 31 am Mittelteil 2 gelagert. Mittels eines Stiftes 32, der durch eine Bohrung im Lager 31 und durch Bohrungen im Zahnsegment 25 steckbar ist, lassen sich die Seitenflügel 3 arretieren.

Wie bereits erwähnt läßt sich das Bestrahlungsgerät optimal an die unterschiedlichen Anatomien von Patienten anpassen, weil der Bestrahlungskopf und seine Seitenflügel um fünf Achsen drehbar und außerdem in der Höhe verstellbar sind. Durch Austausch der Strahlenquellen kann für jeden Patienten die zur Therapie optimale Strahlenquelle eingesetzt werden.

Das Ausführungsbeispiel des erfindungsgemäßen Bestrahlungsgerätes ist so konstruiert, daß es aus seiner Arbeitsposition, die eine Vielzahl von Einstellungen ermöglicht, in eine raumsparende Parkposition gebracht werden kann. Zu diesem Zweck läßt sich der den Bestrahlungskopf tragende Schwenkarm um eine vertikale Achse und der das Gerät stützende Ausleger gleichfalls um eine vertikale Achse um jeweils 90° einklappen.

### Bezugszeichenliste

- 1: Bestrahlungskopf
- 2: Mittelteil des Bestrahlungskopfes
- 3: Seitenflügel
- 4: U-förmiger Bügel
- 5: Drehgelenk
- 6: Schwenkarm
- 7: Drehgelenk
- 8: Säule
- 9: Säulenhalter
- 10: Schaltschrank
- 11: Bedienkonsole
- 12: Rad
- 13: Ausleger
- 14: oberes Gelenkteil
- 15: unteres Gelenkteil
- 16: Aussparung
- 17: Schraube
- 18: Schraubenkopf
- 19: Federelement
- 20: Gewindebohrung
- 21: Gleitfläche
- 22: Ansatz
- 23: Aussparung
- 24: zentrale Öffnung
- 25: Zahnsegment
- 26: Welle
- 27: Schnecke
- 28: Elektromotor
- 29: Zahnrad
- 30: Zahnrad
- 31: Lager
- 32: Stift
- 33: zentraler Kabelkanal des Schwenkarmes
- 34: zentraler Kabelkanal des U-förmigen Bügels
- 35: zentraler Kabelkanal des U-förmigen Bügels
- 36: Anschlagstift
- 37: Scheibe
- A: Achse
- A': Achse
- S: Drehachse
- T: Drehachse
- T': Drehachse
- U: Drehachse
- V: Drehachse
- W: Drehachse
- Z: Drehachse

## Patentansprüche

1. Bestrahlungsgerät zur fotodynamischen Therapie mit einem mit Strahlenquellen ausgerüsteten Bestrahlungskopf (1), welcher aus einem Mittelteil (2) mit zwei an gegenüberliegenden Seiten schwenkbar befestigten Seitenflügeln (3) besteht, sowie mit einer Säule (8) in einem Säulenhalter (9) und einem Schaltschrank (10) wobei das Mittelteil (2) mittels eines ersten Drehgelenkes (5) drehbar an einem Schwenkarm (6) befestigt ist, der mittels eines zweiten Drehgelenkes (7) um eine Achse (W) drehbar an der Säule (8) des am Schaltschrank (10) befestigten Säulenhalters (9) befestigt ist,
**dadurch gekennzeichnet, dass** die Drehachse (U) des ersten Drehgelenkes (5) senkrecht zum Mittelteil (2) steht und die Säule (8) im Säulenhalter (9) entlang ihrer zentralen Achse (Z) verschiebbar und feststellbar geführt und um diese drehbar ist, wobei die Drehachse (U) des Mittelteils (2) und die Drehachse (W) des Schwenkarmes (6) senkrecht zueinander stehen.

2. Bestrahlungsgerät nach Anspruch 1,
**dadurch gekennzeichnet, dass** der Schwenkarm (6) ausziehbar und auf eine vorgebbare Länge einstellbar ist.

3. Bestrahlungsgerät nach Anspruch 1 oder 2,
**dadurch gekennzeichnet, dass** das Mittelteil (2) und die Seitenflügel (3) des Bestrahlungskopfes (1) als rechteckförmige Platten ausgeführt sind, an oder in deren Unterseiten die Strahlenquellen angeordnet sind.

4. Bestrahlungsgerät nach einem der Ansprüche 1 bis 3,
**dadurch gekennzeichnet, dass** die Seitenflügel (3) um mindestens 90° aus der Ebene des Mittelteils (2) schwenkbar sind, so dass der Bestrahlungskopf (1) von einer ebenen Fläche, die vom Mittelteil (2) und den ausgeklappten Seitenflügeln (3) gebildet ist, bis zu einem rechtwinkligen U verstellbar ist, das vom Mittelteil (2) und den um 90° geschwenkten Seitenflügeln (3) gebildet ist.

5. Bestrahlungsgerät nach einem der Ansprüche 1 bis 4,
**dadurch gekennzeichnet, dass** am oberen am Mittelteil (2) drehbar befestigten Ende eines Seitenflügels (3) auf jeder Seite je ein in einer am Mittelteil (2) befestigten Drehachse (A, A') gelagertes Zahnsegment (25) vorgesehen ist, dass eine von einem Elektromotor (28) angetriebene Welle (26) mit je einer Schnecke (27) an jedem Ende der Welle (26) zur Verstellung der Seitenflügel (3) vorgesehen ist, dass die Schnecken (27) gegenläufig sind und dass je eine Schnecke (27) mit einem Zahnsegment (25) kämmt.

6. Bestrahlungsgerät nach einem der Ansprüche 1 bis 4,
**dadurch gekennzeichnet, dass** die Seitenflügel von Hand verstellbar am oberen Ende in einem Lager (31) des Mittelteils (2) gelagert und mittels eines Stiftes (32) im Lager (31) feststellbar sind.

7. Bestrahlungsgerät nach einem der Ansprüche 1 bis 6,
**dadurch gekennzeichnet, dass** das Mittelteil (2) des Bestrahlungskopfes (1) mittels eines U-förmigen Bügels (4) am ersten Drehgelenk (5) befestigt ist.

8. Bestrahlungsgerät nach einem der Ansprüche 1 bis 7,
**dadurch gekennzeichnet, dass** das erste Drehgelenk (5) aus einem unteren Gelenkteil (15) und einem auf diesem drehbar gelagerten oberen Gelenkteil (14) besteht, dass die beiden Gelenkteile (14, 15) mittels einer mit dem oberen Gelenkteil (14) verschraubten und im unteren Gelenkteil (15) gelagerten Scheibe (37) axial fest aber beschränkt verdrehbar verbunden sind und dass die Scheibe (37) eine kreissegmentförmige Aussparung (16) aufweist, in welche ein am unteren Gelenkteil (15) vorgesehener Stift (36) als Anschlag eingreift.

9. Bestrahlungsgerät nach Anspruch8,
**dadurch gekennzeichnet, dass** zur Zentrierung und zur axialen Fixierung an den aufeinanderliegenden Enden der Gelenkteile (14, 15) an einem Gelenkteil (15) ein Ansatz (22) angeordnet ist, während am anderen Gelenkteil (14) eine zum Ansatz (22) korrespondierende Aussparung (23) vorgesehen ist.

10. Bestrahlungsgerät nach Anspruch 8,
**dadurch gekennzeichnet, dass** die Scheibe (37) mit dem oberen Gelenkteil (14) mit Schrauben (17) verbunden ist und mit ihrem äußeren Rand reibschlüssig an einem die Aussparung (23) des unteren Gelenkteiles (15) begrenzenden Kragenteil anliegt und dass mindestens bei einer der Schrauben (17) zwischen dem Schraubenkopf (18) und der Scheibe (15) ein Federelement (19) zur Einstellung des Reibmomentes vorgesehen ist.

11. Bestrahlungsgerät nach Anspruch 10,
**dadurch gekennzeichnet, dass** das Federelement (19) eine Schrauben- (19) oder Tellerfeder ist.

12. Bestrahlungsgerät nach einem der Ansprüche 8 bis 11,
**dadurch gekennzeichnet, dass** elektrische Versorgungsleitungen zur Stromversorgung der Strahlenquellen durch eine zentrale Öffnung des zweiten Drehgelenkes (7), einen zentralen Kabelkanal (33) des Schwenkarmes (6), eine zentrale Öffnung (24) des ersten Drehgelenkes (5) und zwei zentrale Kabelkanäle (34, 35) des U-förmigen Bügels (4) führbar sind.

13. Bestrahlungsgerät nach einem der Ansprüche 1 bis 12,
**dadurch gekennzeichnet, dass** die Strahlenquellen austauschbar sind.

14. Bestrahlungsgerät nach einem der Ansprüche 1 bis 13,
**dadurch gekennzeichnet, dass** als Strahlenquellen Leuchtmittel vorgesehen sind.

15. Bestrahlungsgerät nach Anspruch 14,
**dadurch gekennzeichnet, dass** für die Leuchtmittel Stab- oder Kompaktleuchten vorgesehen sind.

16. Bestrahlungsgerät nach Anspruch 14 oder 15,
**dadurch gekennzeichnet, dass** Leuchtmittel mit einem Spektrum von Ultraviolett über sichtbares Licht bis zu Infrarot vorgesehen sind.

17. Bestrahlungsgerät nach einem der Ansprüche 1 bis 16,
**dadurch gekennzeichnet, dass** der Schaltschrank (10) mittels an seiner Unterseite vorgesehener Räder (12) oder Rollen fahrbar ist.

18. Bestrahlungsgerät nach einem der Ansprüche 1 bis 17,
**dadurch gekennzeichnet, dass** am Schaltschrank (10) zur Standsicherheit ein ausschwenkbarer oder ausziehbarer Ausleger (13) vorgesehen ist.

19. Bestrahlungsgerät nach einem der Ansprüche 1 bis 19,
**dadurch gekennzeichnet, dass** auf der Oberseite des Schaltschranks (10) eine Bedienkonsole (11) vorgesehen ist.

20. Bestrahlungsgerät nach einem der Ansprüche 1 bis 19,
**dadurch gekennzeichnet, dass** zur Kühlung der Strahlenquellen eine Kühlvorrichtung vorgesehen ist.

21. Bestrahlungsgerät nach einem der Ansprüche 1 bis 20,
**dadurch gekennzeichnet, dass** der den Bestrahlungskopf (1) tragende Schwenkarm (6) um die vertikale Achse (Z) und der Ausleger (13) um die vertikale Achse (S) in eine raumsparende Parkposition jeweils um 90° einklappbar sind.

## Claims

1. Irradiation device for photodynamic therapy, comprising an irradiation head (1) equipped with radiation sources, said irradiation head consisting of a central part (2) with two side wings (3) pivotably attached on opposite sides, and also comprising a column (8) in a column support (9) and a switch cabinet (10), wherein the central part (2) is rotatably attached by means of a first rotary joint (5) to a pivot arm (6) which is attached to the column (8) of the column support (9) such that it can rotate about an axis (W) by means of a second rotary joint (7), said column support being attached to the switch cabinet (10), **characterised in that** the axis of rotation (U) of the first rotary joint (5) is perpendicular to the central part (2), and the column (8) in the column support (9) is guided such that it can be displaced and fixed along its central axis (Z) and can be rotated about the latter, wherein the axis of rotation (U) of the central part (2) and the axis of rotation (W) of the pivot arm (6) are perpendicular to one another.

2. Irradiation device according to Claim 1, **characterised in that** the pivot arm (6) can be pulled out and adjusted to a predefinable length.

3. Irradiation device according to Claim 1 or 2, **characterised in that** the central part (2) and the side wings (3) of the irradiation head (1) are designed as rectangular plates, on or in the undersides of which the radiation sources are arranged.

4. Irradiation device according to one of Claims 1 to 3, **characterised in that** the side wings (3) can be pivoted by at least 90° out of the plane of the central part (2), so that the irradiation head (1) can be adjusted from a flat surface, which is formed by the central part (2) and the extended side wings (3), to a right-angled U-shape, which is formed by the central part (2) and the side wings (3) that have been pivoted by 90°.

5. Irradiation device according to one of Claims 1 to 4, **characterised in that** a toothed segment (25) which is mounted on a rotation axle (A, A') attached to the central part (2) is provided on each side at the upper end of a side wing (3) which is rotatably attached to the central part (2), **in that** a shaft (26) driven by an electric motor (28) is provided with a respective worm (27) at each end of the shaft (26) in order to adjust the side wings (3), **in that** the worms (27) move in opposite directions, and **in that** each worm (27) meshes with a respective toothed segment (25).

6. Irradiation device according to one of Claims 1 to 4, **characterised in that** the side wings are mounted at the upper end in a bearing (31) of the central part (2) in such a way that they can be manually adjusted, and can be fixed in the bearing (31) by means of a pin (32).

7. Irradiation device according to one of Claims 1 to 6, **characterised in that** the central part (2) of the irradiation head (1) is attached to the first rotary joint (5) by means of a U-shaped bracket (4).

8. Irradiation device according to one of Claims 1 to 7, **characterised in that** the first rotary joint (5) consists of a lower joint part (15) and an upper joint part (14) which is rotatably mounted thereon, **in that** the two joint parts (14, 15) are connected, in such a way that they are axially fixed but can rotate to a limited extent, by means of a disc (37) which is screwed to the upper joint part (14) and is mounted in the lower joint part (15), and **in that** the disc (37) has a circular-segment-shaped cutout (16) in which a pin (36) engages as a stop, said pin being provided on the lower joint part (15) .

9. Irradiation device according to Claim 8, **characterised in that**, for the purpose of centring and axial fixing at the ends of the joint parts (14, 15) which bear against one another, a protrusion (22) is arranged on one joint part (15) while a cutout (23) which corresponds to the protrusion (22) is provided on the other joint part (14).

10. Irradiation device according to Claim 8 **characterised in that** the disk (37) is connected to the upper joint part (14) by screws (17) and bears with its outer edge in a friction fit against a collar part which delimits the cutout (23) of the lower joint part (15), and **in that** a spring element (19) for adjusting the friction torque is provided on at least one of the screws (17) between the screw head (18) and the disc (37).

11. Irradiation device according to Claim 10, **characterised in that** the spring element (19) is a helical spring (19) or plate spring.

12. Irradiation device according to one of Claims 8 to 11, **characterised in that** electric lines for supplying power to the radiation sources can be passed through a central opening of the second rotary joint (7), a central cable duct (33) of the pivot arm (6), a central opening (24) of the first rotary joint (5), and two central cable ducts (34, 35) of the U-shaped bracket (4).

13. Irradiation device according to one of Claims 1 to 12, **characterised in that** the radiation sources are exchangeable.

14. Irradiation device according to one of Claims 1 to 13, **characterised in that** light generators are provided as radiation sources.

15. Irradiation device according to Claim 14, **characterised in that** strip lamps or compact lamps are provided for the light generators.

16. Irradiation device according to Claim 14 or 15, **characterised in that** light generators with a spectrum from ultraviolet, through visible light, to infrared are provided.

17. Irradiation device according to one of Claims 1 to 16, **characterised in that** the switch cabinet (10) can be moved by means of wheels (12) or rollers provided on its underside.

18. Irradiation device according to one of Claims 1 to 17, **characterised in that** an extension arm (13) which can be pivoted out or pulled out for stabilisation purposes is provided on the switch cabinet (10).

19. Irradiation device according to one of Claims 1 to 19, **characterised in that** an operating console (11) is provided on the upper side of the switch cabinet (10).

20. Irradiation device according to one of Claims 1 to 19, **characterised in that** a cooling device is provided for cooling the radiation sources.

21. Irradiation device according to one of Claims 1 to 20, **characterised in that** the pivot arm (6) which carries the irradiation, head (1) can be folded in about the vertical axis (Z) and the extension arm (13) can be folded in about the vertical axis (S), in each case through 90°, into a space-saving parked position.

## Revendications

1. Dispositif d'irradiation pour la thérapie photodynamique avec une tête d'irradiation (1) équipée de sources de rayonnement et constituée d'un élément central (2) avec deux ailes latérales (3) fixées de manière orientable sur des côtés opposés, ainsi que d'une colonne (8) dans un support de colonne (9) et une armoire électrique (10), dans lequel l'élément central (2) est fixé de manière pivotante au moyen d'une première articulation tournante (5) sur un bras orientable (6) fixé de manière pivotante autour d'un axe (W) au moyen d'une deuxième articulation tournante (7) sur la colonne (8) du support de colonne (9) fixé sur l'armoire électrique (10),
**caractérisé en ce que**
l'axe de rotation (U) de la première articulation tournante (5) est perpendiculaire à l'élément central (2) et la colonne (8) est guidée dans le support de colonne (9) de manière coulissante et verrouillable le long de son axe central (Z) et peut pivoter autour de celui-ci, l'axe de rotation (U) de l'élément central (2) et l'axe de rotation (W) du bras orientable (6) étant perpendiculaires entre eux.

2. Dispositif d'irradiation selon la revendication 1,
**caractérisé en ce que**
le bras orientable (6) peut être étiré et réglé à une longueur prédéfinie.

3. Dispositif d'irradiation selon la revendication 1 ou 2,
**caractérisé en ce que**
l'élément central (2) et les ailes latérales (3) de la tête d'irradiation (1) sont réalisés comme des plateaux rectangulaires dont les faces inférieures comportent les sources de rayonnement.

4. Dispositif d'irradiation selon l'une des revendications 1 à 3,
**caractérisé en ce que**
les ailes latérales (3) sont inclinables sur au moins 90° à partir du plan de l'élément central (2), de sorte que la tête d'irradiation (1) peut être réglée pour passer d'une surface plane formée de l'élément central (2) et des ailes latérales (3) déployées, à un U à angles droit qui est formé de l'élément central (2) est des ailes latérales (3) inclinées à 90°.

5. Dispositif d'irradiation selon l'une des revendications 1 à 4,
**caractérisé en ce que**
l'extrémité supérieure d'une aile latérale (3) fixée de manière pivotante à l'élément central (2), comporte de chaque côté un segment denté (25) monté dans un axe de rotation (A, A') fixé à l'élément central (2), et un arbre (26) entraîné par un moteur électrique (28), avec un pas de vis (27) à chaque extrémité de l'arbre (26) permet de régler les ailes latérales (3), les pas de vis (27) étant inversés et chaque pas de vis (27) s'engrenant avec un segment denté (25).

6. Dispositif d'irradiation selon l'une des revendications 1 à 4,
**caractérisé en ce que**
les ailes latérales sont montées de manière réglable manuellement à l'extrémité supérieure dans un palier (31) de l'élément central (2) et peuvent être fixées au moyen d'une goupille (32) dans le palier (31).

7. Dispositif d'irradiation selon l'une des revendications 1 à 6,
**caractérisé en ce que**
l'élément central (2) de la tête d'irradiation (1) est fixé au moyen d'un étrier en forme de U (4) sur la première articulation tournante (5).

8. Dispositif d'irradiation selon l'une des revendications 1 à 7,
**caractérisé en ce que**
la première articulation tournante (5) est constituée d'un élément d'articulation inférieur (15) et d'un élément d'articulation supérieur (14) pivotant sur celui-ci, les deux éléments d'articulation (14, 15) sont reliés de manière fixe axialement mais pivotante de manière limitée au moyen d'un plaque (37) vissée avec l'élément d'articulation supérieur (14) et logée dans l'élément d'articulation inférieur (15) et la plaque (37) présente un évidement en forme de segment de cercle (16) dans lequel pénètre une goupille (36) prévue en guise de butée sur l'élément d'articulation inférieur (15).

9. Dispositif d'irradiation selon la revendication 8,
**caractérisé en ce que**
pour le centrage et la fixation axiale aux extrémités superposées des éléments d'articulation (14, 15), un pion (22) est monté sur un élément d'articulation (15), tandis qu'un évidement (23) correspondant au pion (22) est prévu sur l'autre élément d'articulation (14).

10. Dispositif d'irradiation selon la revendication 8,
**caractérisé en ce que**
la plaque (37) est reliée à l'élément d'articulation supérieur (14) avec des vis (17) et frotte avec son bord extérieur contre un collet délimitant l'évidement (23) de l'élément d'articulation inférieur (15), et un élément de ressort (19) pour régler le couple de friction est prévu au moins au niveau d'une des vis (17), entre la tête de vis (18) et la plaque (15).

11. Dispositif d'irradiation selon la revendication 10,
**caractérisé en ce que**
l'élément de ressort (19) est un ressort à boudin (19) ou une rondelle-ressort.

12. Dispositif d'irradiation selon l'une des revendications 8 à 11,
**caractérisé en ce que**
des lignes électriques pour l'alimentation des sources de rayonnement peuvent être guidées à travers une ouverture centrale de la deuxième articulation tournante (7), une conduite de câbles centrale (33) du bras orientable (6), une ouverture centrale (24) de la première articulation tournante (5) et deux conduites de câbles centrales (34, 35) de l'étrier en forme de U (4).

13. Dispositif d'irradiation selon l'une des revendications 8 à 12,
**caractérisé en ce que**
les sources de rayonnement sont remplaçables.

14. Dispositif d'irradiation selon l'une des revendications 8 à 13,
**caractérisé en ce que**
des moyens d'éclairage sont prévus en guise de sources de rayonnement.

15. Dispositif d'irradiation selon la revendication 14,
**caractérisé en ce que**
des lampes torches ou compactes sont prévues pour les moyens d'éclairage.

16. Dispositif d'irradiation selon la revendication 14 ou 15,
**caractérisé en ce que**
des moyens d'éclairage sont prévus avec un spectre allant de l'ultraviolet à l'infrarouge en passant par la lumière visible.

17. Dispositif d'irradiation selon l'une des revendications 1 à 16,
**caractérisé en ce que**
l'armoire électrique est mobile au moyen de roues (12) ou de galets prévus sur sa face inférieure.

18. Dispositif d'irradiation selon l'une des revendications 1 à 17,
**caractérisé en ce qu'**
une patte pivotante ou extensible (13) est prévue sur l'armoire électrique (10) pour assurer la stabilité.

19. Dispositif d'irradiation selon l'une des revendications 1 à 18,
**caractérisé en ce qu'**
un pupitre de commande (11) est prévu sur la face supérieure de l'armoire électrique (10).

20. Dispositif d'irradiation selon l'une des revendications 1 à 19,
**caractérisé en ce qu'**
un dispositif de refroidissement est prévu pour refroidir les sources de rayonnement.

21. Dispositif d'irradiation selon l'une des revendications 1 à 20,
**caractérisé en ce que**,
dans une position de rangement peu encombrante, le bras orientable (6) portant la tête d'irradiation (1) et la patte (13) peuvent être rabattus à 90°, respectivement autour de l'axe vertical (Z) et de l'axe vertical (S).
